# EUROPEAN PATENT SPECIFICATION

(11) **EP 2 937 696 B1**
(45) Date of publication and mention of the grant of the patent: **06.06.2018**
(21) Application number: 13865260.7
(22) Date of filing: 18.12.2013
(51) Int. Cl.: G01N 33/574

(54) **METHOD FOR DETECTING PROSTATIC BASAL CELLS**
VERFAHREN ZUR ERKENNUNG VON PROSTATABASALZELLEN
MÉTHODE DE DÉTECTION DANS LES CELLULES BASALES DE LA PROSTATE

(30) Priority: 20.12.2012 JP 2012277980
(43) Date of publication of application: 28.10.2015
(73) Proprietor: National University Corporation Hokkaido University, Sapporo-shi, Hokkaido 060-0808 (JP)
(72) Inventor: HATAKEYAMA, Shigetsugu, Sapporo-shi Hokkaido 060-0808 (JP); TANAKA, Shinya, Sapporo-shi Hokkaido 060-0808 (JP)
(74) Representative: Cabinet Plasseraud
(86) International application number: PCT/JP2013/083915
(87) International publication number: WO 2014/098135

(56) References cited:
- WO-A1-2006/080597
- WO-A2-2014/144657
- JP-A- 2008 528 024
- JP-A- 2012 502 283
- JP-A- 2012 525 404
- ROY BLUM ET AL: "Molecular Signatures of the Primitive Prostate Stem Cell Niche Reveal Novel Mesenchymal-Epithelial Signaling Pathways", PLOS ONE, vol. 5, no. 9, 30 September 2010 (2010-09-30), page e13024, XP055286540, DOI: 10.1371/journal.pone.0013024 & Roy Blum: "Molecular signatures of the primitive prostate stem cell niche reveal novel mesenchymal-epithelial signaling pathways: Supplementary Table S1", Plos One, 2010, volume 5, issue 9, 30 September 2010 (2010-09-30), pages 1-2, XP055286558, Retrieved from the Internet: URL:http://journals.plos.org/plosone/artic le?id=10.1371/journal.pone.0013024 [retrieved on 2016-07-07]
- YOSHIMASA KOSAKA ET AL: "Tripartite Motif-Containing 29 (TRIM29) Is a Novel Marker for Lymph Node Metastasis in Gastric Cancer", ANNALS OF SURGICAL ONCOLOGY, SPRINGER-VERLAG, NE, vol. 14, no. 9, 28 June 2007 (2007-06-28), pages 2543-2549, XP019522879, ISSN: 1534-4681, DOI: 10.1245/S10434-007-9461-1
- JASON R. ROCK ET AL: "Basal cells as stem cells of the mouse trachea and human airway epithelium.", PNAS, VOL. 106(31), 1 January 2009 (2009-01-01), pages 12771-12775, XP055080161, & Jason Rock: "Table S4. Selected genes from the 627 genes significantly upregulated in lectin+,KRT5-GFPhi tracheal BCs.", PNAS vol.106(31), 4 August 2009 (2009-08-04), pages 1-1, XP055286655, Retrieved from the Internet: URL:http://www.pnas.org/content/106/31/127 71.full.pdf?with-ds=yes [retrieved on 2016-07-07]
- Gladell P Paner ET AL: "CAP Laboratory Improvement Programs Best Practice in Diagnostic Immunohistochemistry Prostate Carcinoma and Its Mimics in Needle Core Biopsies", , 1 September 2008 (2008-09-01), XP055284406, Retrieved from the Internet: URL:http://www.archivesofpathology.org/doi /pdf/10.1043/1543-2165(2008)132[1388:BPIDI P]2.0.CO;2 [retrieved on 2016-06-29]
- MOON-KYUN CHO: "Tripartite Motif-Containing 29 Expression in Squamous Cell Carcinoma", ANNALS OF DERMATOLOGY, vol. 24, no. 4, 1 January 2012 (2012-01-01), page 491, XP055285003, KR ISSN: 1013-9087, DOI: 10.5021/ad.2012.24.4.491
- YUKIKO KANNO ET AL: "TRIM29 as a novel prostate basal cell marker for diagnosis of prostate cancer", ACTA HISTOCHEMICA, vol. 116, no. 5, 1 June 2014 (2014-06-01), pages 708-712, XP055284144, AMSTERDAM, NL ISSN: 0065-1281, DOI: 10.1016/j.acthis.2013.12.009
- SCHLOMM T ET AL.: 'Extraction and processing of high quality RNA from impalpable and macroscopically invisible prostate cancer for microarray gene expression analysis.' INT J ONCOL. vol. 27, no. 3, September 2005, pages 713 - 720, XP008077729
- SCHLOMM T ET AL.: 'Marked gene transcript level alterations occur early during radical prostatectomy.' EUR UROL. vol. 53, no. 2, February 2008, pages 333 - 346, XP022396817

## Description

### Technical Field

The present invention relates to a method for detecting prostatic basal cells, a method for identifying the presence, decrease, and disappearance of prostatic basal cells. The present invention enables a reliable detection of prostatic basal cells. The present invention also enables a reliable definite diagnosis of prostate cancer.

### Background Art

The normal prostate consists of glandular cells and basal cells, and a histological picture is observed, in which the basal cells are present around the glandular cells.

Androgen receptors, cytokeratin 8, and cytokeratin 18 are specifically expressed in the glandular cells, and most prostate cancers express androgen receptors, cytokeratin 8, or cytokeratin 18 as markers for these glandular cells. Thus, prostate cancer is considered to be derived from the glandular cells.

The basal cells are known to disappear in prostate cancer. Thus, a definite diagnosis of prostate cancer is made by performing the immunohistostaining of prostate using an antibody to p63 or cytokeratin expressed in normal basal cells and identifying the disappearance of the basal cells.

### Citation List

### Patent Literature

Patent Literature 1: International Publication No. WO 2008/150512
Patent Literature 2: International Publication No. WO 2005/014781
Patent Literature 3: International Publication No. WO 2004/018711
Patent Literature 4: International Publication No. WO 2006/080597
Patent Literature 5: International Publication No. WO 2004/018711

Roy et al (PLOS ONE, 2010, vol. 5, issue 9, pages 1-2) reports the differential gene expression of cells from urogenital sinus of 16-day old murine embryos, mainly urogenital sinus epithelium (UGE) vs urogenital sinus epithelium mesenchyme (UGM). Kosaka et al (Annals of Surgical Oncology, 2007, 14(9): 2543-2549) reports the increased mRNA expression of TRIM29 in gastric cancer tumor tissue. WO2006/080597 further reports diagnostic markers of lung cancer comprising TRIM29. Paner et al (URL: http://www.archivesofpathology.org/doi/pdf/10.1043/1543-2165(2008)132[1388 : BPIDIP] 2.0.CO;2) Rock et al (PNAS, 2009, vol 106, no 31 pp 12771-12775) reports differential gene expression in murine tracheal basal cells. Furthermore, T. Ernst, et. al. discloses the reduced gene expression of TRIM29 in prostate cancer (American Journal of Pathology, Vol.160(6), June 2002, pages 2169-2180).

### Summary of Invention

### Technical Problem

Regarding the above immunohistostaining of basal cells, it is sometimes difficult to determine whether the basal cells have disappeared, only by immunohistostaining using an anti-p63 antibody or anti-cytokeratin antibody.

Thus, an object of the present invention is to provide a method for detecting prostatic basal cells by searching for molecules specifically expressed in prostatic basal cells and analyzing the molecules.

### Solution to Problem

As a result of intensive studies on molecules specifically expressed in basal cells of normal prostate tissue, the present inventors have surprisingly found that tripartite motif-containing protein 29 (TRIM29) is specifically expressed in the basal cells of normal prostate tissue. Then, it has been shown that the basal cells can be detected by visualizing the expression of TRIM29 in the prostate tissue by means of immunohistostaining.

The present invention is based on these findings.

Specifically, the present invention relates to:
[1] An in vitro method for detecting prostatic basal cells, comprising visualizing the expression of tripartite motif-containing protein 29 (TRIM29) protein in basal cells of the prostate by immunohistostaining;
[2] The method for detecting prostatic basal cells according to [1], comprising analyzing the morphology of the glandular tissue structure of the prostate and the visualized expression of tripartite motif-containing protein 29 (TRIM29) protein;
[3] The method for detecting prostatic basal cells according to [1] or [2], further comprising visualizing the expression of cytokeratin and/or p63 protein by immunohistostaining;
[4] The method for detecting prostatic basal cells according to [3], comprising analyzing the morphology of the glandular tissue structure of the prostate and the visualized expression of cytokeratin protein and/or the visualized expression of p63 protein;
[5] The method of any of [1] to [4], wherein the presence, decrease or disappearance of prostatic basal cells is visualized in the prostate tissue collected from the subject as compared to a normal prostate tissue.
[6] A method for diagnosing prostate cancer, comprising a step of detecting prostatic basal cells in prostate tissue collected from a subject by immunohistostaining using an anti-TRIM29 antibody or an antigen-binding fragment thereof;
[7] The diagnostic method according to [6], wherein the decrease or disappearance of prostatic basal cells as compared to those in normal prostate tissue indicates the presence of prostate cancer;
[8] Use of an anti-TRIM29 antibody or an antigen-binding fragment thereof in the in vitro diagnosis of prostate cancer via the detection of TRIM29 in prostatic basal cells.

The gene of TRIM29 is reported to be associated with lung cancer, ovarian serous papillary adenocarcinoma, and cervical intraepithelial neoplasia (Patent Literatures 1 to 4). A method for predicting prognosis for prostate cancer by examining 80 mRNAs comprising TRIM29 is also disclosed (Patent Literature 5). However, it is not reported that TRIM29 is expressed in basal cells of the normal prostate.

### Advantageous Effects of Invention

The method for detecting prostatic basal cells according to the present invention can reliably detect normal prostatic basal cells and is useful for histological studies of the prostate. The combination of the method with the immunohistostaining of expressed cytokeratin and/or p63 can more reliably detect prostatic basal cells.

The method for detecting prostatic basal cells according to the present invention and the method for identifying the presence, decrease, or disappearance of prostatic basal cells according to the present invention enable a definite diagnosis of prostate cancer. The combination of the methods with the immunohistostaining of expressed cytokeratin and/or p63 enables a more reliable definite diagnosis of prostate cancer.

### Brief Description of Drawings

[Figure 1] Figure 1 is a series of micrographs, each of which shows normal prostate tissue or prostate cancer tissue subjected to the immunohistostaining with anti-TRIM29 polyclonal antibodies or an anti-cytokeratin monoclonal antibody (34βE12).
[Figure 2] Figure 2 is a pair of micrographs, each of which shows normal prostate tissue subjected to the immunohistostaining with an anti-TRIM29 monoclonal antibody.
[Figure 3] Figure 3 is a pair of micrographs, each of which shows normal prostate tissue subjected to the immunohistostaining with an anti-TRIM29 monoclonal antibody or an anti-cytokeratin monoclonal antibody (34βE12).

### Description of Embodiments

### [1] Method for Detecting Prostatic Basal Cells

The method for detecting prostatic basal cells according to the present invention comprises visualizing the expression of tripartite motif-containing protein 29 (TRIM29) protein in basal cells of the prostate by immunohistostaining. According to the method, prostate-derived cells expressing TRIM29 can be determined to be basal cells based on their morphology and protein expression. In addition, the method enables the detection of basal cells which form part of the glandular tissue structure by identifying the morphology of the glandular tissue structure of the prostate and the visualized expression of TRIM29 protein.

The animal species having prostatic basal cells which may be detected by the detection method of the present invention is not limited as long as it has basal cells in the prostate; and examples of the animal species include mammals, such as humans, monkeys, dogs, cats, ferrets, cows, horses, goats, sheep, guinea pigs, hamsters, jirds, mice, or rats. The detection method of the present invention can also be used in separated cells. For example, the method can also be applied to primary cultured cells or passage cells comprising separated basal cells from any of the above mammals.

### <Prostate>

Prostate is a gland in the male reproductive system and is located beneath the bladder and in front of the rectum. The prostate is composed of two types of cells: basal cells and glandular cells. Histologically, prostatic basal cells are present around prostatic glandular cells.

### (Prostatic Glandular Cells)

Prostatic glandular cells specifically express androgen receptors, cytokeratin 8, and cytokeratin 18, and most prostate cancers are derived from prostatic glandular cells.

### (Prostatic Basal Cells)

The prostatic basal cells which may be detected by the detection method of the present invention are not limited as long as the cells express TRIM29. However, this does not exclude the presence of prostatic basal cells not expressing TRIM29.

The basal cells specifically express p63, cytokeratin 5, and cytokeratin 14 in addition to TRIM29. p63 is considered to play an important role in the development of the prostate; the nuclei of the basal cells are stained by immunohistostaining with 4A4 which is an anti-p63 monoclonal antibody. Cytokeratin 5 and cytokeratin 14 are present in the cytoplasm and stained by immunohistostaining with 34βE12 which is an anti-cytokeratin monoclonal antibody. The monoclonal antibody 34βE12 is an antibody exhibiting positive reactions to cytokeratin 1 and cytokeratin 10 in addition to cytokeratin 5 and cytokeratin 14.

### <TRIM29>

Tripartite motif-containing protein 29 (TRIM29) belongs to the TRIM gene family, and is also known as ATDC (ataxia-telangiectasia group D-associated protein). Human TRIM29 consists of 588 amino acids. TRIM29 has a plurality of zinc finger motifs and a leucine zipper motif and binds to DNA. TRIM29 is considered to possibly function as a transcriptional regulator in differentiation processes. TRIM29 is known to be associated with ataxia-telangiectasia, and has been pointed out to be associated with the function of suppressing radiation sensitivity.

According to the present disclosure, the base sequence of a nucleic acid encoding analyzable human TRIM29 protein (hereinafter also referred to as human TRIM29 gene) is shown in SEQ ID NO: 1, and the amino acid sequence of the human TRIM29 protein is shown in SEQ ID NO: 2. However, the gene to be analyzed is not limited to the TRIM29 gene having the base sequence as shown in SEQ ID NO: 1 as long as it is expressed in prostatic basal cells and can hybridize to a probe or a primer capable of binding to the TRIM29 gene having the base sequence of SEQ ID NO: 1. The protein to be analyzed is also not limited to the TRIM29 protein having the amino acid sequence as shown in SEQ ID NO: 2 as long as it is expressed in prostatic basal cells and can bind to an antibody capable of binding to the protein having the amino acid sequence of SEQ ID NO: 2. In other words, according to the present invention, the TRIM29 to be analyzed may be TRIM29 having a mutation or mutations as long as it is expressed in prostatic basal cells. It is also not limited to human TRIM29 and may be mammalian TRIM29 including, for example, the TRIM29 of humans, monkeys, dogs, cats, ferrets, cows, horses, goats, sheep, guinea pigs, hamsters, jirds, mice, or rats. Examples of TRIM29 protein having a mutation or mutations include, when human TRIM29 is taken for instance, a protein consisting of an amino acid sequence in which 1 to several, e.g., 1, 2, 3, 4, 5, 6, 7, 8, or 9, amino acids are substituted, deleted, inserted, or added in the amino acid sequence of SEQ ID NO: 2, and a protein consisting of an amino acid sequence having 80% or more sequence identity, e.g., 80% or more, 85% or more, 90% or more, 95% or more, or 98% or more sequence identity, with the amino acid sequence of SEQ ID NO: 2.

### <TRIM29 Protein Expression Analysis>

The TRIM29 expression analysis by immunohistostaining according to the detection method of the present invention involves, but is not limited to, visualizing the expression of TRIM29 protein. By the visualization of the expression of TRIM29 protein, prostatic basal cells can be morphologically identified in the same basal cells, and the morphologies of prostatic basal cells and the glandular tissue structure of the prostate can be identified based on the expression of TRIM29 protein. In other words, the detection method of the present invention preferably involves analyzing, in basal cells which form part of the glandular tissue structure, the morphology of the glandular tissue structure of the prostate and the visualized expression of TRIM29 protein.

As used herein, the "visualization" does not only mean the gross identification of coloring or fluorescence resulting from immunohistostaining through a light microscope or a fluorescence microscope, but includes, for example, the mechanical detection of coloring, fluorescence, luminescence, or radiation (i.e., a signal) followed by the identification of a picture or the like obtained by imaging the signal.

As used herein, the "analyzing the morphology of the glandular tissue structure of the prostate and the visualized expression of the tripartite motif-containing protein 29 (TRIM29) protein" includes not only the simultaneous observation of the morphology of the glandular tissue structure of the prostate and the expression of TRIM29 protein, but also, for example, the observation of the morphology of the glandular tissue structure of the prostate in comparison with the picture or the like obtained by imaging the signal.

The analysis of TRIM29 protein in prostatic basal cells is carried out by immunohistostaining. This is because the position of basal cells in the prostate tissue or the morphology of basal cells can be identified by immunohistostaining.

### (Immunohistostaining)

Immunohistostaining is an established technique and can be carried out based on a known method except for the use of an antibody specific for TRIM29. In other words, immunohistostaining is a method which involves detecting a specific antigen expressed in cells of tissue slices by using an antibody specifically recognizing the antigen.

Specifically, immunohistostaining can be carried out, for example, as follows. A prostate tissue is frozen and sliced, or a fixed, paraffin-embedded tissue block is sliced to prepare tissue slices. An antibody recognizing TRIM29 is reacted with the surface of the tissue slice. The anti-TRIM29 antibody can be labeled with a substance emitting a signal to detect TRIM29 protein on each tissue slice. Alternatively, a second antibody to the anti-TRIM29 antibody may be labeled with a substance emitting a signal without labeling the anti-TRIM29 antibody. In addition, biotin may be bound to the anti-TRIM29 antibody or the second antibody, followed by labeling avidin, specifically binding to biotin, with a substance emitting a signal.

The substance which may be used for labeling the antibody includes, but is not limited to, a fluorescent dye (e.g., rhodamine, fluorescein isothiocyanate (FITC), or a rare earth metal chelate), a radioactive substance (e.g., ³H, ¹⁴C, or ¹²⁵I), or an enzyme (e.g., peroxidase, alkaline phosphatase, or β-D-galactosidase). The detection of a signal can be carried out using, but not limited to, fluorescence, radiation (autoradiography), luminescence, and coloring. For example, when rhodamine, or FITC is used as the fluorescent dye, the signal can be detected using a fluorescence microscope. For example, when alkaline phosphatase is used and coloring is performed using NBT/BCIP, the signal can be detected under a light microscope and the morphology of cells can simultaneously be observed.

Specifically, commonly used methods include a peroxidase anti-peroxidase method (PAP method), a streptavidin-biotin complex method (sABC method), a polymer reagent method in which a secondary antibody and a labeling enzyme are bound to a polymer, and a method using a primary antibody labeled with FITC or using an HRP-labeled anti-FITC antibody as a secondary antibody.

### (Anti-TRIM29 Antibody)

The anti-TRIM29 antibody used for the immunohistostaining according to the present invention can be prepared by a known method except for using TRIM29 protein or a partial peptide thereof as an immunogen. The anti-TRIM29 antibody may be polyclonal antibodies or a monoclonal antibody; and a monoclonal antibody is preferable. This is because a monoclonal antibody often produces less cross-reaction with other proteins. A known or commercially available antibody can also be used.

For example, a monoclonal antibody can be prepared according to the method of Koehler and Milstein (Nature 256: 495-497, 1975). For polyclonal antibodies, for example, an antigen bound to BSA or KLH can be mixed with an adjuvant, such as Freund's complete adjuvant, and intracutaneously immunized into a rabbit on a periodic basis, followed by collecting blood once the antibody titer in the blood has elevated, and the collected blood can be used as an antiserum or the antibodies can be purified by a known method before use.

The antibody used in the immunological analysis method may also be an antibody fragment containing an antigen-binding site for TRIM29 protein (antigen-binding fragment). Examples of the antibody fragment include F(ab')₂, Fab', Fab, and Fv. These antibody fragments can each be obtained, for example, by digesting an antibody with a proteinase (for example, pepsin or papain) by a conventional method and subsequently purifying the resultant by a conventional method for separating and purifying a protein.

In the method for detecting prostatic basal cells according to the present invention, it is preferable to perform the expression analysis of cytokeratin protein and/or p63 protein in addition to the TRIM29 protein expression analysis. In other words, TRIM29 protein and cytokeratin protein may be analyzed; TRIM29 protein and p63 protein may be analyzed; or the three proteins may be analyzed.

According to the present embodiment, 2 or more proteins may be analyzed in the same tissue slice from the same subject (patient). In addition, 2 or more proteins may be separately analyzed in each of different tissue slices from the same subject (patient).

### <Cytokeratin Protein Expression Analysis>

The cytokeratin expression analysis by immunohistostaining in the detection method of the present invention involves, but is not limited to, visualizing the expression of cytokeratin protein. By the visualization of the expression of cytokeratin protein, prostatic basal cells can be morphologically identified in the same basal cells, and the morphologies of prostatic basal cells and the glandular tissue structure of the prostate can be identified based on the expression of cytokeratin protein. In other words, the detection method of the present invention preferably involves analyzing the morphology of the glandular tissue structure of the prostate and the visualized expression of cytokeratin protein in basal cells which form part of the glandular tissue structure.

The expression analysis of cytokeratin protein can be carried out based on a known method except for using an antibody specific for cytokeratin. Specifically, the analysis can be carried out according to the method described in "TRIM29 Protein Expression Analysis" except for using an antibody specific for cytokeratin. The amino acid sequence of cytokeratin 5 is shown in SEQ ID NO: 4, and the amino acid sequence of cytokeratin 14 is shown in SEQ ID NO: 6. The base sequence encoding cytokeratin 5 is shown in SEQ ID NO: 3 and the base sequence encoding cytokeratin 14 is shown in SEQ ID NO: 5.

The antibody specific for cytokeratin may be polyclonal antibodies or a monoclonal antibody; and a monoclonal antibody is preferable. This is because a monoclonal antibody often produces less cross-reaction with other proteins. A known or commercially available antibody can also be used. For example, 34βE12 monoclonal antibody can be used, which is a commercially available anti-cytokeratin monoclonal antibody; this antibody recognizes cytokeratins 1, 5, 10, and 14.

### <p63 Protein Expression Analysis>

The p63 expression analysis in the detection method of the present invention involves, but is not limited to, visualizing the expression of p63 protein. By the visualization of the expression of p63 protein, prostatic basal cells can be morphologically identified in the same basal cells, and the morphologies of prostatic basal cells and the glandular tissue structure of the prostate can be identified based on the expression of p63 protein. In other words, the detection method in the present invention preferably involves analyzing the morphology of the glandular tissue structure of the prostate and the visualized expression of p63 protein in basal cells which form part of the glandular tissue structure.

The expression analysis of p63 protein can be carried out based on a known method except for using an antibody specific for p63. Specifically, the analysis can be carried out according to the method described in "TRIM29 Protein Expression Analysis" except for using an antibody specific for p63. The amino acid sequence of p63 is shown in SEQ ID NO: 8 and the base sequence encoding p63 is shown in SEQ ID NO: 7.

The antibody specific for p63 may be polyclonal antibodies or a monoclonal antibody; and a monoclonal antibody is preferable. This is because a monoclonal antibody often produces less cross-reaction with other proteins. A known or commercially available antibody can also be used. For example, 4A4 monoclonal antibody can be used, which is a commercially available anti-p63 monoclonal antibody.

### [2] Method for Identifying Presence, Decrease, or Disappearance of Prostatic Basal Cells

The method for identifying the presence, decrease, or disappearance of prostatic basal cells according to the present invention comprises visualizing the expression of tripartite motif-containing protein 29 (TRIM29) protein in the prostate tissue by means of immunohistostaining using the method for detecting prostatic basal cells according to the present invention. In the identification method, the expression of TRIM29 protein is detected. The detection of the expression of TRIM29 protein can be carried out in the same manner as described in "TRIM29 Protein Expression Analysis" in the method for detecting prostatic basal cells according to the present invention. After detecting prostatic basal cells, the presence, decrease, or disappearance of the prostatic basal cells is identified.

In the method for identifying the presence, decrease, or disappearance of prostatic basal cells according to the present invention, the visualization of the expression of TRIM29 protein by immunohistostaining enables the observation of the relation between the morphologies of the basal cells and the glandular tissue structure of the prostate and the expression of TRIM29 protein, enabling the reliable identification of the presence, decrease, or disappearance of prostatic basal cells which form part of the glandular tissue structure.

For example, when the presence, decrease, or disappearance of prostatic basal cells is identified on tissue sections, the "presence," "decrease," or "disappearance" of TRIM29 protein in a test sample can be visually determined under a fluorescence microscope or a light microscope based on the visualized expression level of TRIM29 protein as compared to the expression level of TRIM29 protein in the basal cells of the normal prostate tissue. A signal, such as coloring, luminescence, fluorescence, or radiation, in an image taken with a camera or the like can be mechanically visualized to analyze the expression level of TRIM29 protein. Such a method enables the determination of the "presence," "decrease," or "disappearance" of TRIM29 protein in a test sample by seeing the image with the naked eye and also enables the determination of the "presence," "decrease," or "disappearance" of TRIM29 protein by the mechanical digitization of the signal.

For example, when the level of TRIM29 protein in a test sample is comparable as compared to the expression level of TRIM29 protein in basal cells of the normal prostate tissue, prostatic basal cells can be determined to be "present" in the test sample. When little TRIM29 protein can be detected in a test sample , prostatic basal cells can be determined to "disappear." When the level of TRIM29 protein in a test sample is intermediate between those for "presence" and "disappearance," prostatic basal cells can be determined to "decrease."

### <Prostate Cancer>

In prostate cancer, prostatic basal cells completely disappear, or decrease with some cells remaining. Thus, the method for detecting prostatic basal cells and the method for identifying the presence, decrease, and disappearance of prostatic basal cells according to the present invention enable a diagnosis or definite diagnosis of prostate cancer.

### [3] Immunohistostaining Kit

The immunohistostaining kit for detecting prostatic basal cells according to the present disclosure comprises an anti-TRIM29 antibody. The immunohistostaining kit of the present disclosure can be used in the method for detecting prostatic basal cells, the method for identifying the presence, decrease, or disappearance of prostatic basal cells, and a method for diagnosing prostate cancer.

The immunohistostaining kit of the present disclosure preferably comprises an antibody specifically binding to TRIM29 or a fragment having its antigen-binding site (antigen-binding fragment) in a desired form. The anti-TRIM29 antibody used here may be the anti-TRIM29 antibody described in "[1] Method for Detecting Prostatic Basal Cells." In other words, the antibody may be a monoclonal antibody or polyclonal antibodies. The antibody fragment is not particularly limited as long as it has the ability to specifically bind to TRIM29, i.e., it is a fragment having the antigen-binding site (antigen-binding fragment). The antibody fragment used here may be, for example, Fab, Fab', F(ab')₂, or Fv.

In addition, the immunohistostaining kit for detecting prostatic basal cells according to the present disclosure preferably comprises an anti-cytokeratin antibody and/or an anti-p63 antibody. The anti-cytokeratin antibody and the anti-p63 antibody used here may also be the anti-cytokeratin antibody and anti-p63 antibody described in "[1] Method for Detecting Prostatic Basal Cells," or fragments having their antigen-binding sites (antigen-binding fragments).

In addition, the immunohistostaining kit for detecting prostatic basal cells according to the present disclosure may comprise an enzyme such as peroxidase, alkaline phosphatase, β-D-galactosidase, and glucose oxidase. The kit may also comprise, for example, fluorescein isothiocyanate or a rare earth metal chelate as a fluorescent substance. The kit may also comprise a radioactive isotope such as ³H, ¹⁴C, and ¹²⁵I. The kit according to the present invention may use other labeling substances such as biotin, avidin, and a chemiluminescent substance. The kit of the present disclosure may comprise, for example, a suitable substrate for the enzyme or chemiluminescent substance.

The kit of the present disclosure may comprise instructions stating that it is for the detection of prostatic basal cells. The description to the effect that it is for the detection of prostatic basal cells may be presented on a container for the kit. It may be stated that the kit is used for the diagnosis or definite diagnosis of prostate cancer.

The anti-TRIM29 antibody can be used for the manufacture of the immunohistostaining kit for detecting prostatic basal cells. The anti-cytokeratin antibody can also be used for the manufacture of the immunohistostaining kit for detecting prostatic basal cells. In addition, the anti-p63 antibody can be used for the manufacture of the immunohistostaining kit for detecting prostatic basal cells.

### Examples

The present invention is specifically described below with reference to examples.

### <Example 1>

In this Example, the immunohistostaining of prostate tissues (normal and cancer) was carried out using an anti-TRIM29 antibody. The immunohistostaining was performed using prostate specimens of 16 samples stored and managed in Department of Cancer Pathology, Hokkaido University Graduate School of Medicine. The profiles of the prostate specimens provided for analysis are shown in Table 1. Fifteen samples having prostate cancer and 1 sample having prostatic intraepithelial neoplasia (PIN) were analyzed.

### [Table 1]

**Table 1. Clinicopathologic Findings in 15 Cases having Prostate Cancer**

| | ***n* (%)** |
|---|---|
| Age (years) | |
| <65 | 2 (13.3) |
| 65-75 | 7 (46.7) |
| >75 | 6 (40) |
| PSA (ng/ml) | |
| 4<PSA<6 | 5 (33.3) |
| 6≦PSA<10 | 8 (53.3) |
| 10< | 2 (13.3) |

| T (Extent of Primary Tumor) | |
|---|---|
| T1c | 12(80) |
| T2a | 2 (13.3) |
| T2b | 0 (0) |
| T2c | 1 (6.7) |

| N (Lymph Node Metastasis) | |
|---|---|
| N0 | 15 (100.0) |
| N1< | 0 (0) |

| M (Distant Metastasis) | |
|---|---|
| M0 | 15 (100) |
| M1< | 0 (0) |

| TNM Stage | |
|---|---|
| I | 0 (0) |
| II | 15 (100) |
| III | 0 (0) |
| IV | 0 (0) |

### (1) Staining with Anti-TRIM29 Rabbit Polyclonal Antibodies

An anti-TRIM29 rabbit polyclonal antibodies (ATDC(H-300)SC-33151 manufactured by Santa Cruz) as a primary antibody were diluted 200 times with a diluent (PBS containing 1% BSA) and added to each tissue slice. The primary reaction was carried out at 37°C for 30 minutes. The resultant tissue slices were washed 3 times with a wash solution (PBS), and a reagent containing a peroxidase-labeled anti-rabbit antibody (Dako REAL™ Envision™ Detection Reagent peroxidase rabbit/mouse) as a secondary antibody was added to the tissue slice. The second reaction was carried out at room temperature for 30 minutes. The resultant was washed 3 times with the wash solution and then subjected to coloring reaction using a coloring solution (REAL™ DAB + CHROMOGEN from Dako).

### (2) Staining with Anti-cytokeratin Monoclonal Antibody

In addition, the immunohistostaining of prostate tissues (normal and cancer) was carried out using an anti-cytokeratin monoclonal antibody, 34βE12, as a primary antibody. The steps described in the above (1) were repeated except for using 34βE12 (H1205 Nichirei anti-polymer cytokeratin monoclonal antibody) diluted 2 times with 1% BSA in place of the anti-TRIM29 rabbit polyclonal antibody diluted 200 times.

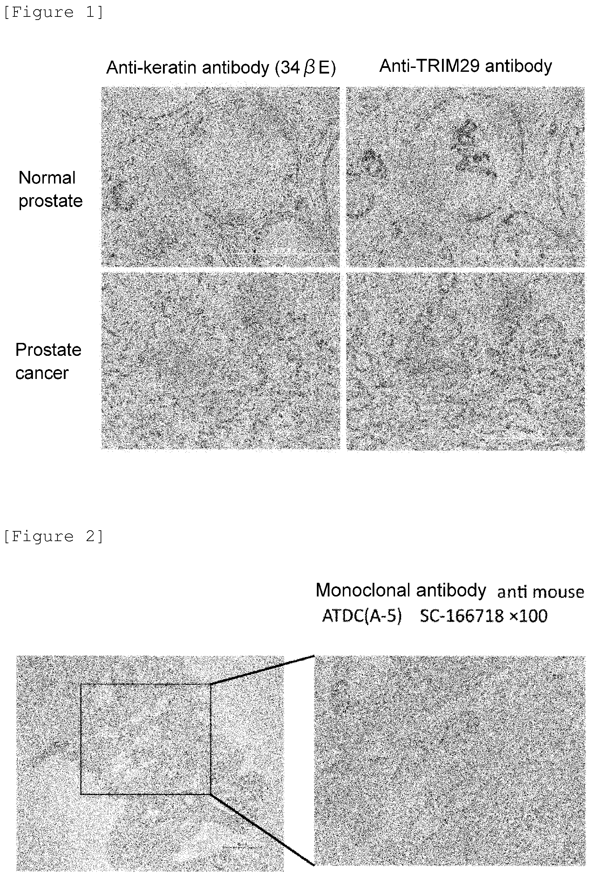

The results of immunohistostaining as described in the above (1) and (2) are shown in Figure 1. It turned out that the anti-TRIM29 antibody specifically stained basal cells present around glandular cells in the normal prostate tissue. It turned out that cells and tissue stained by the anti-TRIM29 antibody decreased or disappeared in the prostate cancer tissue. These results were comparable to those obtained when immunohistostaining was carried out using 34βE12. The combined use of 34βE12 and the anti-TRIM29 antibody can increase the reliability of the diagnosis of prostate cancer.

### <Example 2>

In this Example, the immunohistostaining of normal prostate was carried out using an anti-TRIM29 mouse monoclonal antibody (anti-ATDC (A-5) antibody) (sc-166718) as a primary antibody.

The steps described in subsection (1) of Example 1 were repeated except that the anti-TRIM29 mouse monoclonal antibody A-5 was used as a primary antibody, that an anti-mouse IgG antibody was used as a secondary antibody, and that only normal prostate tissue was used as a sample.

As shown in Figure 2, non-specific staining was not observed and basal cells were clearly stained.

### <Example 3>

In this Example, the immunohistostaining of normal prostate was carried out using an anti-TRIM29 mouse monoclonal antibody (anti-ATDC (A-5) antibody) (sc-166718) and an anti-cytokeratin monoclonal antibody.

The steps described in Example 2 were repeated for the anti-TRIM29 mouse monoclonal antibody, and the steps described in subsection (2) of Example 1 were repeated for the anti-cytokeratin monoclonal antibody except for using only normal prostate tissue.

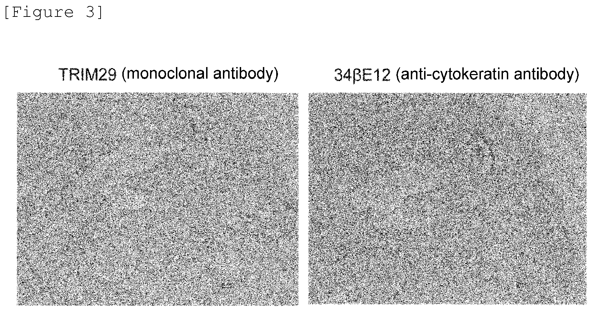

As shown in Figure 3, the staining with the anti-TRIM29 mouse monoclonal antibody provided a result very similar to that of the staining with the anti-cytokeratin monoclonal antibody; and the combination of these two immunohistostainings enabled the reliable identification of prostatic basal cells.

When the results of immunohistostaining in Examples 1 to 3 were analyzed, the expression of TRIM29 was found to disappear in cancer tissue portions for all cases of prostate cancer. In contrast, for prostatic intraepithelial neoplasia, the expression of TRIM29 was observed in basal cell portions as with the normal prostate. These results are shown in Table 2.

### [Table 2]

**Table 2. Degree of TRIM29 Staining in Prostate Cancer**

| | | Percentage of TRIM29-Positive Acini in All Acini | | | |
|---|---|---|---|---|---|
| Gleason Score | | **0** | **0-30** | **30-60** | **60-100** |
| Gleason score | 3 + 3 = 6 (n=8) | 8 | 0 | 0 | 0 |
| Gleason score | 3 + 4 = 7 (n=3) | 3 | 0 | 0 | 0 |
| Gleason score | 4 + 3 = 7 (n=1) | 1 | 0 | 0 | 0 |
| Gleason score | 4 + 4 = 8 (n=2) | 2 | 0 | 0 | 0 |
| Gleason score | 4 + 5 = 9 (n=1) | 1 | 0 | 0 | 0 |
| PIN (n=1) | | 0 | 0 | 0 | 1 |
| Normal site (n=15) | | 0 | 0 | 0 | 15 |

### Industrial Applicability

The method for detecting prostatic basal cells according to the present invention can be used for the histological study of prostate. The method for identifying the presence, decrease, or disappearance of prostatic basal cells according to the present invention enables a definite diagnosis of prostate cancer. In addition, the combination of the methods with the expression analysis of cytokeratin enables a more reliable definite diagnosis of prostate cancer.

## Claims

1. An in vitro method for detecting prostatic basal cells, comprising visualizing the expression of tripartite motif-containing protein 29 (TRIM29) protein in basal cells of a prostate by immunohistostaining.

2. The method for detecting prostatic basal cells according to claim 1, comprising analyzing the morphology of the glandular tissue structure of the prostate and the visualized expression of tripartite motif-containing protein 29 (TRIM29) protein.

3. The method for detecting prostatic basal cells according to claim 1 or 2, further comprising visualizing the expression of cytokeratin and/or p63 protein by immunohistostaining.

4. The method for detecting prostatic basal cells according to claim 3, comprising analyzing the morphology of the glandular tissue structure of the prostate and the visualized expression of cytokeratin protein and/or the visualized expression of p63 protein.

5. The method of any one of Claims 1 to 4, wherein the presence, decrease or disappearance of prostatic basal cells is visualized in the prostate tissue collected from the subject as compared to a normal prostate tissue.

6. A method for diagnosing prostate cancer, comprising a step of detecting prostatic basal cells in prostate tissue collected from a subject by immunohistostaining using an anti-TRIM29 antibody or an antigen-binding fragment thereof.

7. The diagnostic method according to claim 6, wherein the decrease or disappearance of prostatic basal cells as compared to those in normal prostate tissue indicates the presence of prostate cancer.

8. Use of an anti-TRIM29 antibody or an antigen-binding fragment thereof in the in vitro diagnosis of prostate cancer via the detection of TRIM29 in prostatic basal cells.

## Patentansprüche

1. Ein In-vitro-Verfahren zum Nachweisen von Prostatabasalzellen, umfassend ein Visualisieren der Expression eines Proteins Tripartite Motif-enthaltendes-Protein 29 (TRIM29) in Basalzellen einer Prostata durch Immunhistofärbung.

2. Das Verfahren zum Nachweisen von Prostatabasalzellen gemäß Anspruch 1, umfassend ein Analysieren der Morphologie der Drüsengewebestruktur der Prostata und der visualisierten Expression des Proteins Tripartite Motif-enthaltendes-Protein 29 (TRIM29).

3. Das Verfahren zum Nachweisen von Prostatabasalzellen gemäß Anspruch 1 oder 2, ferner umfassend ein Visualisieren der Expression von Cytokeratin und/oder p63-Protein durch Immunhistofärbung.

4. Das Verfahren zum Nachweisen von Prostatabasalzellen gemäß Anspruch 3, umfassend ein Analysieren der Morphologie der Drüsengewebestruktur der Prostata und der visualisierten Expression von Cytokeratinprotein und/oder der visualisierten Expression von p63-Protein.

5. Das Verfahren gemäß einem der Ansprüche 1 bis 4, wobei das Vorhandensein, die Abnahme oder das Verschwinden von Prostatabasalzellen in dem vom Subjekt entnommenem Prostatagewebe im Vergleich zu einem normalen Prostatagewebe visualisiert wird.

6. Ein Verfahren zur Diagnose von Prostatakrebs, umfassend einen Schritt des Nachweisens von Prostatabasalzellen in von einem Subjekt entnommenem Prostatagewebe durch Immunhistofärbung unter Verwendung eines Anti-TRIM29-Antikörpers oder eines antigen-bindenden Fragments davon.

7. Das diagnostische Verfahren gemäß Anspruch 6, wobei die Abnahme oder das Verschwinden von Prostatabasalzellen im Vergleich zu jenen in normalem Prostatagewebe das Vorhandensein von Prostatakrebs anzeigt.

8. Eine Verwendung eines Anti-TRIM29-Antikörpers oder eines antigen-bindenden Fragments davon bei der In-vitro-Diagnose von Prostatakrebs über das Nachweisen von TRIM29 in Prostatabasalzellen.

## Revendications

1. Procédé *in vitro* de détection de cellules basales prostatiques, comprenant la visualisation de l'expression d'une protéine 29 contenant un motif tripartite (TRIM29) dans des cellules basales d'une prostate par immunohistocoloration.

2. Procédé de détection de cellules basales prostatiques selon la revendication 1, comprenant l'analyse de la morphologie de la structure de tissu glandulaire de la prostate et de l'expression visualisée de la protéine 29 contenant un motif tripartite (TRIM29).

3. Procédé de détection de cellules basales prostatiques selon la revendication 1 ou 2, comprenant en outre la visualisation de l'expression de la cytokératine et/ou de la protéine p63 par immunohistocoloration.

4. Procédé de détection de cellules basales prostatiques selon la revendication 3, comprenant l'analyse de la morphologie de la structure de tissu glandulaire de la prostate et de l'expression visualisée de la protéine de cytokératine et/ou de l'expression visualisée de la protéine p63.

5. Procédé selon l'une quelconque des revendications 1 à 4, dans lequel la présence, la diminution ou la disparition de cellules basales prostatiques est visualisée dans le tissu de prostate recueilli auprès du sujet et comparée à un tissu de prostate normal.

6. Procédé de diagnostic d'un cancer de la prostate, comprenant une étape de détection de cellules basales prostatiques dans un tissu de prostate recueilli auprès d'un sujet par immunohistocoloration à l'aide d'un anticorps anti-TRIM29 ou d'un fragment de liaison à l'antigène de celui-ci.

7. Procédé de diagnostic selon la revendication 6, dans lequel la diminution ou la disparition de cellules basales prostatiques comparée à celles dans un tissu de prostate normal indique la présence d'un cancer de la prostate.

8. Utilisation d'un anticorps anti-TRIM29 ou d'un fragment de liaison à l'antigène de celui-ci dans le diagnostic *in vitro* d'un cancer de la prostate via la détection de TRIM29 dans des cellules basales prostatiques.


### SEQUENCE LISTING

<110> NATIONAL UNIVERSITY CORPORATION HOKKAIDO UNIVERSITY
<120> A method for detecting basal cells of postate gland
<130> 671812/P2012-103-WO01/LP0056
<150> JP 2012-277980
   <151> 2012-12-20
<160> 8
<170> PatentIn version 3.1
<210> 1
   <211> 1767
   <212> DNA
   <213> Homo sapiens
<400> 1
   atggaagctg cagatgcctc caggagcaac gggtcgagcc cagaagccag ggatgcccgg      60
   agcccgtcgg gccccagtgg cagcctggag aatggcacca aggctgacgg caaggatgcc     120
   aagaccacca acgggcacgg cggggaggca gctgagggca agagcctggg cagcgccctg     180
   aagccagggg aaggtaggag cgccctgttc gcgggcaatg agtggcggcg acccatcatc     240
   cagtttgtcg agtccgggga cgacaagaac tccaactact tcagcatgga ctctatggaa     300
   ggcaagaggt cgccgtacgc agggctccag ctgggggctg ccaagaagcc acccgttacc     360
   tttgccgaaa agggcgagct gcgcaagtcc attttctcgg agtcccggaa gcccacggtg     420
   tccatcatgg agcccgggga gacccggcgg aacagctacc cccgggccga cacgggcctt     480
   ttttcacggt ccaagtccgg ctccgaggag gtgctgtgcg actcctgcat cggcaacaag     540
   cagaaggcgg tcaagtcctg cctggtgtgc caggcctcct tctgcgagct gcatctcaag     600
   ccccacctgg agggcgccgc cttccgagac caccagctgc tcgagcccat ccgggacttt     660
   gaggcccgca agtgtcccgt gcatggcaag acgatggagc tcttctgcca gaccgaccag     720
   acctgcatct gctacctttg catgttccag gagcacaaga atcatagcac cgtgacagtg     780
   gaggaggcca aggccgagaa ggagacggag ctgtcattgc aaaaggagca gctgcagctc     840
   aagatcattg agattgagga tgaagctgag aagtggcaga aggagaagga ccgcatcaag     900
   agcttcacca ccaatgagaa ggccatcctg gagcagaact tccgggacct ggtgcgggac     960
   ctggagaagc aaaaggagga agtgagggct gcgctggagc agcgggagca ggatgctgtg    1020
   gaccaagtga aggtgatcat ggatgctctg gatgagagag ccaaggtgct gcatgaggac    1080
   aagcagaccc gggagcagct gcatagcatc agcgactctg tgttgtttct gcaggaattt    1140
   ggtgcattga tgagcaatta ctctctcccc ccacccctgc ccacctatca tgtcctgctg    1200
   gagggggagg gcctgggaca gtcactaggc aacttcaagg acgacctgct caatgtatgc    1260
   atgcgccacg ttgagaagat gtgcaaggcg gacctgagcc gtaacttcat tgagaggaac    1320
   cacatggaga acggtggtga ccatcgctat gtgaacaact acacgaacag cttcgggggt    1380
   gagtggagtg caccggacac catgaagaga tactccatgt acctgacacc caaaggtggg    1440
   gtccggacat cataccagcc ctcgtctcct ggccgcttca ccaaggagac cacccagaag    1500
   aatttcaaca atctctatgg caccaaaggt aactacacct cccgggtctg ggagtactcc    1560
   tccagcattc agaactctga caatgacctg cccgtcgtcc aaggcagctc ctccttctcc    1620
   ctgaaaggct atccctccct catgcggagc caaagcccca aggcccagcc ccagacttgg    1680
   aaatctggca agcagactat gctgtctcac taccggccat tctacgtcaa caaaggcaac    1740
   gggattgggt ccaacgaagc cccatga                                         1767
<210> 2
   <211> 588
   <212> PRT
   <213> Homo sapiens
<400> 2
   Met Glu Ala Ala Asp Ala Ser Arg Ser Asn Gly Ser Ser Pro Glu Ala
   1               5                   10                  15
   Arg Asp Ala Arg Ser Pro Ser Gly Pro Ser Gly Ser Leu Glu Asn Gly
               20                  25                  30
   Thr Lys Ala Asp Gly Lys Asp Ala Lys Thr Thr Asn Gly His Gly Gly
           35                  40                  45
   Glu Ala Ala Glu Gly Lys Ser Leu Gly Ser Ala Leu Lys Pro Gly Glu
       50                  55                  60
   Gly Arg Ser Ala Leu Phe Ala Gly Asn Glu Trp Arg Arg Pro Ile Ile
   65                  70                  75                  80
   Gln Phe Val Glu Ser Gly Asp Asp Lys Asn Ser Asn Tyr Phe Ser Met
                   85                  90                  95
   Asp Ser Met Glu Gly Lys Arg Ser Pro Tyr Ala Gly Leu Gln Leu Gly
               100                 105                 110
   Ala Ala Lys Lys Pro Pro Val Thr Phe Ala Glu Lys Gly Glu Leu Arg
           115                 120                 125
   Lys Ser Ile Phe Ser Glu Ser Arg Lys Pro Thr Val Ser Ile Met Glu
       130                 135                 140
   Pro Gly Glu Thr Arg Arg Asn Ser Tyr Pro Arg Ala Asp Thr Gly Leu
   145 150 155 160
   Phe Ser Arg Ser Lys Ser Gly Ser Glu Glu Val Leu Cys Asp Ser Cys
   165 170 175
   Ile Gly Asn Lys Gln Lys Ala Val Lys Ser Cys Leu Val Cys Gln Ala
   180 185 190
   Ser Phe Cys Glu Leu His Leu Lys Pro His Leu Glu Gly Ala Ala Phe
   195 200 205
   Arg Asp His Gln Leu Leu Glu Pro Ile Arg Asp Phe Glu Ala Arg Lys
   210 215 220
   Cys Pro Val His Gly Lys Thr Met Glu Leu Phe Cys Gln Thr Asp Gln
   225 230 235 240
   Thr Cys Ile Cys Tyr Leu Cys Met Phe Gln Glu His Lys Asn His Ser
   245 250 255
   Thr Val Thr Val Glu Glu Ala Lys Ala Glu Lys Glu Thr Glu Leu Ser
   260 265 270
   Leu Gln Lys Glu Gln Leu Gln Leu Lys Ile Ile Glu Ile Glu Asp Glu
   275 280 285
   Ala Glu Lys Trp Gln Lys Glu Lys Asp Arg Ile Lys Ser Phe Thr Thr
   290 295 300
   Asn Glu Lys Ala Ile Leu Glu Gln Asn Phe Arg Asp Leu Val Arg Asp
   305 310 315 320
   Leu Glu Lys Gln Lys Glu Glu Val Arg Ala Ala Leu Glu Gln Arg Glu
   325 330 335
   Gln Asp Ala Val Asp Gln Val Lys Val Ile Met Asp Ala Leu Asp Glu
   340 345 350
   Arg Ala Lys Val Leu His Glu Asp Lys Gln Thr Arg Glu Gln Leu His
   355 360 365
   Ser Ile Ser Asp Ser Val Leu Phe Leu Gln Glu Phe Gly Ala Leu Met
   370 375 380
   Ser Asn Tyr Ser Leu Pro Pro Pro Leu Pro Thr Tyr His Val Leu Leu
   385 390 395 400
   Glu Gly Glu Gly Leu Gly Gln Ser Leu Gly Asn Phe Lys Asp Asp Leu
                   405                 410                 415
   Leu Asn Val Cys Met Arg His Val Glu Lys Met Cys Lys Ala Asp Leu
               420                 425                 430
   Ser Arg Asn Phe Ile Glu Arg Asn His Met Glu Asn Gly Gly Asp His
           435                 440                 445
   Arg Tyr Val Asn Asn Tyr Thr Asn Ser Phe Gly Gly Glu Trp Ser Ala
       450                 455                 460
   Pro Asp Thr Met Lys Arg Tyr Ser Met Tyr Leu Thr Pro Lys Gly Gly
   465                 470                 475                 480
   Val Arg Thr Ser Tyr Gln Pro Ser Ser Pro Gly Arg Phe Thr Lys Glu
                   485                 490                 495
   Thr Thr Gln Lys Asn Phe Asn Asn Leu Tyr Gly Thr Lys Gly Asn Tyr
               500                 505                 510
   Thr Ser Arg Val Trp Glu Tyr Ser Ser Ser Ile Gln Asn Ser Asp Asn
           515                 520                 525
   Asp Leu Pro Val Val Gln Gly Ser Ser Ser Phe Ser Leu Lys Gly Tyr
       530                 535                 540
   Pro Ser Leu Met Arg Ser Gln Ser Pro Lys Ala Gln Pro Gln Thr Trp
   545                 550                 555                 560
   Lys Ser Gly Lys Gln Thr Met Leu Ser His Tyr Arg Pro Phe Tyr Val
                   565                 570                 575
   Asn Lys Gly Asn Gly Ile Gly Ser Asn Glu Ala Pro
               580                 585
<210> 3
   <211> 1773
   <212> DNA
   <213> Homo sapiens
<400> 3
   atgtctcgcc agtcaagtgt gtccttccgg agcgggggca gtcgtagctt cagcaccgcc     60
   tctgccatca ccccgtctgt ctcccgcacc agcttcacct ccgtgtcccg gtccgggggt    120
   ggcggtggtg gtggcttcgg cagggtcagc cttgcgggtg cttgtggagt gggtggctat    180
   ggcagccgga gcctctacaa cctggggggc tccaagagga tatccatcag cactagtggt    240
   ggcagcttca ggaaccggtt tggtgctggt gctggaggcg gctatggctt tggaggtggt    300
   gccggtagtg gatttggttt cggcggtgga gctggtggtg gctttgggct cggtggcgga    360
   gctggctttg gaggtggctt cggtggccct ggctttcctg tctgccctcc tggaggtatc    420
   caagaggtca ctgtcaacca gagtctcctg actcccctca acctgcaaat cgaccccagc    480
   atccagaggg tgaggaccga ggagcgcgag cagatcaaga ccctcaacaa taagtttgcc    540
   tccttcatcg acaaggtgcg gttcctggag cagcagaaca aggttctgga caccaagtgg    600
   accctgctgc aggagcaggg caccaagact gtgaggcaga acctggagcc gttgttcgag    660
   cagtacatca acaacctcag gaggcagctg gacagcatcg tgggggaacg gggccgcctg    720
   gactcagagc tgagaaacat gcaggacctg gtggaagact tcaagaacaa gtatgaggat    780
   gaaatcaaca agcgtaccac tgctgagaat gagtttgtga tgctgaagaa ggatgtagat    840
   gctgcctaca tgaacaaggt ggagctggag gccaaggttg atgcactgat ggatgagatt    900
   aacttcatga agatgttctt tgatgcggag ctgtcccaga tgcagacgca tgtctctgac    960
   acctcagtgg tcctctccat ggacaacaac cgcaacctgg acctggatag catcatcgct   1020
   gaggtcaagg cccagtatga ggagattgcc aaccgcagcc ggacagaagc cgagtcctgg   1080
   tatcagacca agtatgagga gctgcagcag acagctggcc ggcatggcga tgacctccgc   1140
   aacaccaagc atgagatctc tgagatgaac cggatgatcc agaggctgag agccgagatt   1200
   gacaatgtca agaaacagtg cgccaatctg cagaacgcca ttgcggatgc cgagcagcgt   1260
   ggggagctgg ccctcaagga tgccaggaac aagctggccg agctggagga ggccctgcag   1320
   aaggccaagc aggacatggc ccggctgctg cgtgagtacc aggagctcat gaacaccaag   1380
   ctggccctgg acgtggagat cgccacttac cgcaagctgc tggagggcga ggaatgcaga   1440
   ctcagtggag aaggagttgg accagtcaac atctctgttg tcacaagcag tgtttcctct   1500
   ggatatggca gtggcagtgg ctatggcggt ggcctcggtg gaggtcttgg cggcggcctc   1560
   ggtggaggtc ttgccggagg tagcagtgga agctactact ccagcagcag tgggggtgtc   1620
   ggcctaggtg gtgggctcag tgtggggggc tctggcttca gtgcaagcag tggccgaggg   1680
   ctgggggtgg gctttggcag tggcgggggt agcagctcca gcgtcaaatt tgtctccacc   1740
   acctcctcct cccggaagag cttcaagagc taa                                1773
<210> 4
   <211> 590
   <212> PRT
   <213> Homo sapiens
<400> 4
<210> 5
   <211> 1419
   <212> DNA
   <213> Homo sapiens
<400> 5
   atgaccacct gcagccgcca gttcacctcc tccagctcca tgaagggctc ctgcggcatc 60
   gggggcggca tcgggggcgg ctccagccgc atctcctccg tcctggccgg agggtcctgc 120
   cgcgccccca gcacctacgg gggcggcctg tctgtctcat cctcccgctt ctcctctggg 180
   ggagcctacg ggctgggggg cggctatggc ggtggcttca gcagcagcag cagcagcttt 240
   ggtagtggct ttgggggagg atatggtggt ggccttggtg ctggcttggg tggtggcttt 300
   ggtggtggct ttgctggtgg tgatgggctt ctggtgggca gtgagaaggt gaccatgcag 360
   aacctcaatg accgcctggc ctcctacctg gacaaggtgc gtgctctgga ggaggccaac 420
   gccgacctgg aagtgaagat ccgtgactgg taccagaggc agcggcctgc tgagatcaaa 480
   gactacagtc cctacttcaa gaccattgag gacctgagga acaagattct cacagccaca 540
   gtggacaatg ccaatgtcct tctgcagatt gacaatgccc gtctggccgc ggatgacttc 600
   cgcaccaagt atgagacaga gttgaacctg cgcatgagtg tggaagccga catcaatggc 660
   ctgcgcaggg tgctggacga actgaccctg gccagagctg acctggagat gcagattgag 720
   agcctgaagg aggagctggc ctacctgaag aagaaccacg aggaggagat gaatgccctg 780
   agaggccagg tgggtggaga tgtcaatgtg gagatggacg ctgcacctgg cgtggacctg 840
   agccgcattc tgaacgagat gcgtgaccag tatgagaaga tggcagagaa gaaccgcaag 900
   gatgccgagg aatggttctt caccaagaca gaggagctga accgcgaggt ggccaccaac 960
   agcgagctgg tgcagagcgg caagagcgag atctcggagc tccggcgcac catgcagaac 1020
   ctggagattg agctgcagtc ccagctcagc atgaaagcat ccctggagaa cagcctggag 1080
   gagaccaaag gtcgctactg catgcagctg gcccagatcc aggagatgat tggcagcgtg 1140
   gaggagcagc tggcccagct ccgctgcgag atggagcagc agaaccagga gtacaagatc 1200
   ctgctggacg tgaagacgcg gctggagcag gagatcgcca cctaccgccg cctgctggag 1260
   ggcgaggacg cccacctctc ctcctcccag ttctcctctg gatcgcagtc atccagagat 1320
   gtgacctcct ccagccgcca aatccgcacc aaggtcatgg atgtgcacga tggcaaggtg 1380
   gtgtccaccc acgagcaggt ccttcgcacc aagaactga 1419
<210> 6
   <211> 472
   <212> PRT
   <213> Homo sapiens
<400> 6
   Met Thr Thr Cys Ser Arg Gln Phe Thr Ser Ser Ser Ser Met Lys Gly
   1               5                   10                  15
   Ser Cys Gly Ile Gly Gly Gly Ile Gly Gly Gly Ser Ser Arg Ile Ser
               20                  25                  30
   Ser Val Leu Ala Gly Gly Ser Cys Arg Ala Pro Ser Thr Tyr Gly Gly
           35                  40                  45
   Gly Leu Ser Val Ser Ser Ser Arg Phe Ser Ser Gly Gly Ala Tyr Gly
       50                  55                  60
   Leu Gly Gly Gly Tyr Gly Gly Gly Phe Ser Ser Ser Ser Ser Ser Phe
   65                  70                  75                  80
   Gly Ser Gly Phe Gly Gly Gly Tyr Gly Gly Gly Leu Gly Ala Gly Leu
                   85                  90                  95
   Gly Gly Gly Phe Gly Gly Gly Phe Ala Gly Gly Asp Gly Leu Leu Val
               100                 105                 110
   Gly Ser Glu Lys Val Thr Met Gln Asn Leu Asn Asp Arg Leu Ala Ser
           115                 120                 125
   Tyr Leu Asp Lys Val Arg Ala Leu Glu Glu Ala Asn Ala Asp Leu Glu
       130                 135                 140
   Val Lys Ile Arg Asp Trp Tyr Gln Arg Gln Arg Pro Ala Glu Ile Lys
   145                 150                 155                 160
   Asp Tyr Ser Pro Tyr Phe Lys Thr Ile Glu Asp Leu Arg Asn Lys Ile
                   165                 170                 175
   Leu Thr Ala Thr Val Asp Asn Ala Asn Val Leu Leu Gln Ile Asp Asn
               180                 185                 190
   Ala Arg Leu Ala Ala Asp Asp Phe Arg Thr Lys Tyr Glu Thr Glu Leu
           195                 200                 205
   Asn Leu Arg Met Ser Val Glu Ala Asp Ile Asn Gly Leu Arg Arg Val
       210                 215                 220
   Leu Asp Glu Leu Thr Leu Ala Arg Ala Asp Leu Glu Met Gln Ile Glu
   225                 230                 235                 240
   Ser Leu Lys Glu Glu Leu Ala Tyr Leu Lys Lys Asn His Glu Glu Glu
                   245                 250                 255
   Met Asn Ala Leu Arg Gly Gln Val Gly Gly Asp Val Asn Val Glu Met
               260                 265                 270
   Asp Ala Ala Pro Gly Val Asp Leu Ser Arg Ile Leu Asn Glu Met Arg
           275                 280                 285
   Asp Gln Tyr Glu Lys Met Ala Glu Lys Asn Arg Lys Asp Ala Glu Glu
       290                 295                 300
   Trp Phe Phe Thr Lys Thr Glu Glu Leu Asn Arg Glu Val Ala Thr Asn
   305                 310                 315                 320
   Ser Glu Leu Val Gln Ser Gly Lys Ser Glu Ile Ser Glu Leu Arg Arg
                   325                 330                 335
   Thr Met Gln Asn Leu Glu Ile Glu Leu Gln Ser Gln Leu Ser Met Lys
               340                 345                 350
   Ala Ser Leu Glu Asn Ser Leu Glu Glu Thr Lys Gly Arg Tyr Cys Met
           355                 360                 365
   Gln Leu Ala Gln Ile Gln Glu Met Ile Gly Ser Val Glu Glu Gln Leu
       370                 375                 380
   Ala Gln Leu Arg Cys Glu Met Glu Gln Gln Asn Gln Glu Tyr Lys Ile
   385                 390                 395                 400
   Leu Leu Asp Val Lys Thr Arg Leu Glu Gln Glu Ile Ala Thr Tyr Arg
                   405                 410                 415
   Arg Leu Leu Glu Gly Glu Asp Ala His Leu Ser Ser Ser Gln Phe Ser
               420                 425                 430
   Ser Gly Ser Gln Ser Ser Arg Asp Val Thr Ser Ser Ser Arg Gln Ile
   435 440 445
   Arg Thr Lys Val Met Asp Val His Asp Gly Lys Val Val Ser Thr His
   450 455 460
   Glu Gln Val Leu Arg Thr Lys Asn
   465 470
<210> 7
   <211> 2043
   <212> DNA
   <213> Homo sapiens
<400> 7
   atgaattttg aaacttcacg gtgtgccacc ctacagtact gccctgaccc ttacatccag      60
   cgtttcgtag aaaccccagc tcatttctct tggaaagaaa gttattaccg atccaccatg     120
   tcccagagca cacagacaaa tgaattcctc agtccagagg ttttccagca tatctgggat     180
   tttctggaac agcctatatg ttcagttcag cccattgact tgaactttgt ggatgaacca     240
   tcagaagatg gtgcgacaaa caagattgag attagcatgg actgtatccg catgcaggac     300
   tcggacctga gtgaccccat gtggccacag tacacgaacc tggggctcct gaacagcatg     360
   gaccagcaga ttcagaacgg ctcctcgtcc accagtccct ataacacaga ccacgcgcag     420
   aacagcgtca cggcgccctc gccctacgca cagcccagct ccaccttcga tgctctctct     480
   ccatcacccg ccatcccctc caacaccgac tacccaggcc cgcacagttt cgacgtgtcc     540
   ttccagcagt cgagcaccgc caagtcggcc acctggacgt attccactga actgaagaaa     600
   ctctactgcc aaattgcaaa gacatgcccc atccagatca aggtgatgac cccacctcct     660
   cagggagctg ttatccgcgc catgcctgtc tacaaaaaag ctgagcacgt cacggaggtg     720
   gtgaagcggt gccccaacca tgagctgagc cgtgaattca acgagggaca gattgcccct     780
   cctagtcatt tgattcgagt agaggggaac agccatgccc agtatgtaga agatcccatc     840
   acaggaagac agagtgtgct ggtaccttat gagccacccc aggttggcac tgaattcacg     900
   acagtcttgt acaatttcat gtgtaacagc agttgtgttg gagggatgaa ccgccgtcca     960
   attttaatca ttgttactct ggaaaccaga gatgggcaag tcctgggccg acgctgcttt    1020
   gaggcccgga tctgtgcttg cccaggaaga gacaggaagg cggatgaaga tagcatcaga    1080
   aagcagcaag tttcggacag tacaaagaac ggtgatggta cgaagcgccc gtttcgtcag    1140
   aacacacatg gtatccagat gacatccatc aagaaacgaa gatccccaga tgatgaactg    1200
   ttatacttac cagtgagggg ccgtgagact tatgaaatgc tgttgaagat caaagagtcc    1260
   ctggaactca tgcagtacct tcctcagcac acaattgaaa cgtacaggca acagcaacag    1320
   cagcagcacc agcacttact tcagaaacag acctcaatac agtctccatc ttcatatggt    1380
   aacagctccc cacctctgaa caaaatgaac agcatgaaca agctgccttc tgtgagccag   1440
   cttatcaacc ctcagcagcg caacgccctc actcctacaa ccattcctga tggcatggga   1500
   gccaacattc ccatgatggg cacccacatg ccaatggctg gagacatgaa tggactcagc   1560
   cccacccagg cactccctcc cccactctcc atgccatcca cctcccactg cacaccccca   1620
   cctccgtatc ccacagattg cagcattgtc agtttcttag cgaggttggg ctgttcatca   1680
   tgtctggact atttcacgac ccaggggctg accaccatct atcagattga gcattactcc   1740
   atggatgatc tggcaagtct gaaaatccct gagcaatttc gacatgcgat ctggaagggc   1800
   atcctggacc accggcagct ccacgaattc tcctcccctt ctcatctcct gcggacccca   1860
   agcagtgcct ctacagtcag tgtgggctcc agtgagaccc ggggtgagcg tgttattgat   1920
   gctgtgcgat tcaccctccg ccagaccatc tctttcccac cccgagatga gtggaatgac   1980
   ttcaactttg acatggatgc tcgccgcaat aagcaacagc gcatcaaaga ggagggggag   2040
   tga                                                                 2043
<210> 8
   <211> 680
   <212> PRT
   <213> Homo sapiens
<400> 8